# EUROPÄISCHE PATENTANMELDUNG

(11) **EP 2 418 000 A1**
(43) Veröffentlichungstag der Anmeldung: **15.02.2012**
(21) Anmeldenummer: 09850926.8
(22) Anmeldetag: 29.10.2009
(51) Int. Cl.: A61N 5/06

(54) **VERFAHREN UND VORRICHTUNG ZUR UMWANDLUNG UND ÜBERTRAGUNG EINES ENERGIEINFORMATIONS-SIGNALS AUF EINE PERSON**

(71) Anmelder: Etory Ltd, Mahe (SC)
(72) Erfinder: GERMANOV, Evgeny, Pavlovich, Moscow 129085 (RU); KUTUSHOV, Mikhail Vladimirovich, Moscow 125414 (RU); GRINSHTEIN, Mark, 42141 Natania (IL); SHRAYBMAN, Mikhail, 46390 Herzlia (IL)
(74) Vertreter: Jeck, Anton
(86) Internationale Anmeldenummer: PCT/RU2009/000590
(87) Internationale Veröffentlichungsnummer: WO 2011/053180

(57) **Zusammenfassung**

Die Erfindung betrifft ein Verfahren zur Umwandlung und Übertragung des Energie-Informationssignals. Es sieht den Empfang von einem superschwachen polarisierten Energie-Informationssignals von der Signalquelle vor sowie die Anwendung des genannten Signals zur Einwirkung auf ein biologisches oder anderes Objekt. Dabei wird das Energie-Informationssignal in ein Informationswellen- Bündel umgewandelt und in den kohärenten Zustand gebracht, indem es durch wenigstens einen zwischen zwei Polarisatoren angeordneten Kristall durchgelassen wird, damit das umgewandelte Signal nachfolgend auf das Objekt einwirkt, darunter zwecks unmittelbarer Einwirkung oder zwecks vermittelter Einwirkung unter Einsatz von einem Zwischenträger, welcher der Einwirkung des umgewandelten Energie-Informationssignals ausgesetzt worden ist. Gemäß einzelnen Ausgestaltungen erfolgt die Einwirkung auf ein Objekt, indem es die Einwirkung von einem Träger erlebt, welcher der Einwirkung des umgewandelten Energie-Informationssignals ausgesetzt worden ist. Gemäß einzelnen Ausgestaltungen dient als Quelle des Energie-Informationssignals das biologische Objekt, insbesondere der Körper oder ein Stoff aus folgender Gruppe: histologisches Präparat auf dem Objektglas, Vollblut, Blutplasma oder biologische Flüssigkeit: Harn, Speichel, Sperma, Träne, Schweiß, Hautabdruck und /oder ein Stück von Resektat oder sein Abdruck auf einem beliebigen Material. Der Wandler enthält Kontakt 1, Becher (Behälter) 2 für die Aufnahme des Objektes für die Einwirkung, obere Grundplatte 3, Stütze 4, Folienhalter 5, die obere Polarisationsfolie 6, Quarzplatte 7, Quarzhalter 8, den zweiten Folienhalter 9, die zweite Stütze 10, Justiermitnehmer 11, die untere Polarisationsfolie 12, den unteren Ring 13 des Folienhalters 12, untere Grundplatte 14, die dritte Stütze 15, Dämpfer (4 Stck.) 16, Auflagefläche für die Quelle des Energie-Informationssignals. Somit sind das wirksame Verfahren und Einrichtung zur Verstärkung, Umwandlung und Invertierung des Signals sowohl von biologischen Objekten als auch von den Arzneimitteln, Lebensmitteln und Trägern entwickelt worden. Infolge der Einwirkung durch das umgewandelte Energie-Informationssignal weist das biologische Objekt ein um 25 % kleineres allgemeines biologisches Index (ABI), ein um 20 % kleineres Fotonenindex (FI) und dreifach vergrößerte Anpassungsreserve (AR) auf. Das Produkt weist wenigstens um 25% vergrößerten kennzeichnenden Effektivitätskennzahlen. Dabei ist die Zuverlässigkeit der Informationssignalsverstärkung erhöht, der Arbeitsablauf im Zusammenhang mit Auftragen der Information auf den Träger vereinfacht, die Behandlungsdauer von verschiedenen Pathologien darunter auch Krebs verkürzt.

## Beschreibung

Die Erfindung bezieht sich auf die Biophysik. Sie kann in beliebigen technischen Gebieten und insbesondere in der Medizin und der Medizintechnik, in der Lebensmittel- und Chemieindustrie angewendet werden. Die Erfindung wird getrennt und/oder in Kombination mit dem vegetativen Resonanztest und/oder in Kombination mit der Bioresonanztherapie realisiert.

Der Stand der Technik wird durch Verfahren der Umwandlung und der Übertragung des Energie-Informationssignals der feinen physischen Felder (fine physical fields - FPF) zur Einwirkung auf ein Objekt gekennzeichnet. Das geschieht durch Einrichtungen zur Übertragung der Informationen auf ein Trägerobjekt (zuckerhaltiges Granulat und andere zur Informationsspeicherung geeigneten Stoffe) sowie durch verschiedene Verfahren und Einrichtungen für die Bioresonanztherapie und die vegetativen Resonanztests.

Die bekannten Verfahren und Einrichtungen für die Bioresonanztherapie gehören zur sich entwickelnden Fachrichtung in der Behandlungs- und Vorbeugungsmedizin. Die Geräte für die Bioresonanztherapie arbeiten nach dem Induktionsprinzip mit den dem menschlichen Körper eigenen Schwingungen. Da die dem Patienten kohärenten Schwingungen und Signale von der Natur her energie-informationshaltig sind, können diese drahtgebunden übertragen werden. Diese Tatsache wurde anhand von Versuchen nachgewiesen. Die Ableitung dieser Schwingungen und Signale vom Körper des Patienten erfolgt mit Hilfe von Elektroden über ein Kabel, welches diese an das Gerät weiterleitet. Die Schwingungen geraten in eine Präzisionseinrichtung und werden in Schwingungen mit umgekehrter Polarität umgesetzt. Danach gehen sie in Form von einer verstärkten Information über ein zweites Kabel zum Patienten zurück. Das führt zum Löschen bzw. zur Verringerung des Gehalts an pathologischer Information. Zu Behandlungszwecken wird in den Geräten für Bioresonanztherapie das sog. "Schwingungsmodell des Patienten" angewendet. In diesem Fall wird der Isotherapie-Grundsatz "aequalia aequalibus curentur" benutzt. Jedoch werden als therapeutisches Agens nicht die Substanzen und Materialien des Körpers des Patienten verwendet (Autonosoden, wie es in der "biochemischen" Isotherapie der praxisspezifische Fall ist), sondern seine elektromagnetischen Schwingungen. Das heißt, es geht um ein "biophysisches" Verfahren. Die Bioresonanztherapie wirkt in der biophysischen Ebene als Informationsresonanztherapie. Das grundlegende Prinzip der Bioresonanztherapie ist die Inversion: Die Energie-Informationssignale (gleich - Schwingungen) werden von der Hand des Patienten abgefühlt. Sie kommen zum Eingang des Geräts und werden darin um 180 Grad umgekehrt, verstärkt und vom Ausgang des Geräts aus an die andere Hand des Patienten weitergeleitet. Dabei werden die pathologischen Schwingungen gedämpft (beseitigt), während die normalen physiologischen Schwingungen nur leicht abgeschwächt werden, was als ein Übungseinfluss zu betrachten ist. Einige deutsche Fabrikate für Bioresonanztherapie (z. B. "BICOM", "RITEC 2000" und andere Geräte) enthalten eine sog. Separiereinrichtung (Separator). Der Separator ermöglicht die Trennung von physiologischen und pathologischen Schwingungen. Physiologische Schwingungen kommen unverändert an den Patienten zurück. Die pathologischen Schwingungen werden dagegen umgekehrt, verstärkt und dann für die nachfolgende Behandlung ebenfalls benutzt. Der Separator ist in der Regel eine sehr teuere und technisch komplizierte Einrichtung. Die damit erreichten klinischen Ergebnisse sind dabei nicht viel besser als die Ergebnisse, die ohne Separator gewonnen werden, http://www.npl-rez.ru/litra/brt.html.

Der vegetative Resonanztest erlaubt, den aktuellen allgemeinen Zustand des Patienten zu ermitteln. Das heißt, der vegetative Resonanztest ermöglicht,
- den augenblicklichen Schädigungsgrad des Immunsystems, die virusbedingte, bakterielle, mykotische Belastung des Körpers sowie die Ortung dieser Belastungen in den Körperorganen zu bestimmen,
- die Organe mit verschiedenen Störungen zu erkennen,
- die Effektivität und die Verträglichkeit der Arzneimittel festzustellen,
- die allgemeine allergiebedingte Belastung sowie die Allergene zu ermitteln,
- das Vor- und das Onkologiebild zu offenbaren,
- die Hilfstherapie auszuwählen,
- den Mangel an Vitaminen, Enzymen, Hormonen, Mikroelementen zu ermitteln,
- kystöse Erscheinungen zu entdecken,
- die geistig-seelische Beanspruchung einzuschätzen,
- die Anpassungsfähigkeiten des zentralen Nervensystems zu normalisieren und zu steigern,
- die Anpassungsreserven des Körpers zu bewerten; dabei handelt es sich sowohl um die aktuellen optimalen Anpassungsreserven als auch um die höchstmöglichen Reserven eines konkreten Menschen,
- den Signifikanzgrad von psychologischen Problemen bei der Erschöpfung der Anpassungsreserven und folglich bei der Herausbildung von Pathologie beim konkreten Patienten festzustellen.

Noch mehr: Die Probleme in Organen und Systemen des Körpers lassen sich bereits während der funktionellen Veränderungen erkennen, wenn noch keine physische Organschädigung vorliegt. Folglich kann die Entwicklung der Krankheit verhindert werden. Dieses Verfahren gilt zurzeit als ziemlich präzise.

Zahlreiche Studien haben nachgewiesen: Der Hauptinformationsträger sowohl innerhalb des biologischen Objekts als auch unter einzelnen biologischen Objekten, darunter auch unter Menschen, ist die Energie-Informationsstrahlung der feinen physischen Felder (FPF). Der eigentliche Informationsübertragungsvorgang ist ein Energie-Vorgang, ein räumlicher und zeitlicher Vorgang. Die Studien haben gezeigt, dass die Raum-Zeit-Struktur der Außen-Makrowelt eine ununterbrochen wiederkehrende Reihe von Einwirkungen durchmachte und dadurch in ein chemisches Kontinuum der molekularen Mikrowelt der Lebewesen umgewandet wurde. Dabei trug sie zur Umwandlung der chemischen Gefüge in die Funktionsstrukturen bei. Der lebendige und der leblose Stoff bestehen aus den gleichen Elementen des Periodensystems. Bei der Abgrenzung von lebendigem und leblosem Stoff spielt der Charakter der Struktur eine wichtige Rolle, allerdings nur, wenn die jeweilige Struktur eine bestimmte Funktion hat. Der Übergang vom Leben zum Tod unter Beibehaltung der vorhandenen Struktur und Abbruch der lebensspezifischen (das Leben festlegenden) Funktionen ist nur für den lebendigen Stoff möglich. Die allgemeine Beschaffenheit des Lebendigen ist das Reproduktionsvermögen. Jedoch besteht die kennzeichnende Haupteigenschaft darin, dass jedes Lebewesen seine eigene individuelle Informations-Befehls-Struktur hat, denn die Selbstreproduktion ohne Vererblichkeit von Informationen und Entwicklungsabläufen ist unmöglich.

Die Energie-Informationssignale kommen in den Schaltkreis der Einrichtungen sowohl von den gesunden als auch von den krankheitsbetroffenen Zellen und Geweben. Diese Energie-Informationssignale sind sehr schwach. Die anhand der vorhandenen Geräte durchgeführten Tests sind manchmal nicht imstande, die eine oder andere Pathologie zu erkennen. Deswegen ist der Bedarf an einer solchen Umwandlung, welche die Verstärkung der Informationssignale sicherstellen kann, schon lange herangereift.

Die Wirkungsverstärkung der für Homöopathie geeigneten Präparate wird anhand einer Verdünnung und eines Umschüttelns oder nach dem elektronischen Verfahren mit Hilfe von einem Transfer vorgenommen.

Jedoch weisen alle oben genannten Methoden ihre offenen und verdeckten Mängel auf. Zu den Hauptmängeln zählen: schwache Signale, die von den kranken Organen und Systemen kommen, sowie schwache Signale, die von den Arzneimitteln an die Informationsträger übertragen werden. Darüber hinaus haben die genannten Verfahren kein gutes Vermögen zur Verstärkung der Energie-Informationssignale.

Das vorgeschlagene Verfahren zur Verstärkung der Energie-Informationssignale ist einfach und zuverlässig. Es ermöglicht die Bestimmung von sehr schwachen Energie-Informationssignalen und die Verstärkung dieser Signale in solchen Systemen, bei denen superschwache Felder zur Diagnose und zur Herstellung von Arzneimitteln eingesetzt werden.

Bekanntlich sind sowohl das ganze Lebewesen als auch alle seine Systeme Quellen von schwachen (physiologischen oder harmonischen) Schwingungen innerhalb von einem breiten Frequenzbereich. Die Krankheitserreger haben auch ihre eigenen Frequenzen. Wird der Körper von einer Krankheit betroffen, so entstehen darin neue Schwingungsquellen. Es geht dabei um die sog. "pathologischen" oder "disharmonischen" Schwingungen, welche das physiologische Gleichgewicht verletzen.

Der bekannten Methode des vegetativen Resonanztests liegt eine genau festgestellte Tatsache zugrunde: Alle biologischen Lebensformen haben ihr einzigartiges, d. h. nur diesen jeweiligen Lebensformen eigenes spezifisches Schwingungsfrequenzspektrum. Der menschliche Frequenzbereich beträgt 1520 bis 9460 kHz. Der Frequenzbereich der pathogenen Organismen (Pilze, Viren, Helminthen, Milben) liegt innerhalb von 77 bis 900 kHz. Folglich wurden alle inhärenten Frequenzgänge der Parasiten, Pilze und Viren artspezifisch genau erkannt und aufgenommen.

Ein weiterer ähnlicher Stand der Technik ist die Sondierung des Gegenstands mit einem gleichläufigen oder konischen (divergenten) Lichtbündel. Im ersten Fall werden dabei Parallelprojektionen und im zweiten Fall konische Projektionen gebildet.

Folgende Variationen der Sondierung sind möglich:
- Drehung des gestrahlten Bündels während der Sondierung um das unbewegliche Objekt,
- Drehung (Neigung) des Objekts zum unbeweglichen gestrahlten Bündel während der Sondierung.

Die Schemata mit dem drehbaren Bündel und dem unbeweglichen Objekt unterscheiden sich je nach der Laufbahn, welche das Sondierungsbündel in Bezug auf das Objekt umschreibt, oder die Fläche (Raster), wo die Projektionen erfasst werden. Folgende Ansätze sind bekannt:
- außeraxiale drehbare feine Lochblende in der Ebene einer Aperturblende des Mikroobjektivs. Sondierungsbahn - Kreislinie;
- eindimensionale Verschiebung einer punktförmigen Quelle in der Ebene der Aperturblende des Kondensators und Erzeugung einer Schrägbeleuchtung. Sondierungsbahn - Gerade (1 D);
- Einsatz eines Mikrospiegelmusters DMD (Digital Micromirror Devices). Die Mikrospiegel werden in der Aperturblende des Mikroobjektivs angeordnet, um eine schräge Beleuchtung zu bekommen. Dieses Schema kann nur für kleine schräge Beleuchtung zu bekommen. Dieses Schema kann nur für kleine Objekte angewendet werden. Sondierungsbahn - beliebig, zweidimensional (2D);
- zweidimensionale Verschiebung einer punktförmigen Quelle in der Ebene der Aperturblende des Mikroobjektivs und Erzeugung einer schrägen Beleuchtung. Sondierungsbahn - beliebig, zweidimensional (2D).

Unter diesen Schemata sind solche zu bevorzugen, welche eine zweidimensionale Bahn sicherstellen.

Alle Ansätze haben einen gemeinsamen Nachteil: Der Winkel bei der Sondierung des Objekts ist durch die numerische Apertur des Mikroobjektivs begrenzt und beträgt max. 90ε. (Patent RU 2140661 "Verfahren für konfokale dreidimensionale Abtastmikroskopie und konfokales Tomographie-Abtastmikroskop" G.G. Levin, G.N. Vischnyakov, F. V. Bulygin, 1999).

Jedoch kann auch dieses Verfahren kein erwartetes Ergebnis zur Verstärkung des Energie-Informationssignals im Schaltkreis (ohne Schaltkreis) für Bioresonanztherapie und vegetativen Resonanztest hervorbringen.

Bekanntlich wird die Strahlung im optischen Bereich während des Durchgangs durch die Polarisatoren und die Kristalle "gefärbt". Bei einer Null-Anisotropie der Brechungszahl Dn oder bei einer Null-Stärke L des einachsigen Mediums zwischen den Polarisatoren (das heißt mit einfachen Worten, bei fehlendem Medium oder Isotropie) ist der Durchlässigkeitsgrad eines solchen Systems strikt gleich Null, T = 0. Die Anisotropie des Phasenverlaufs im Kristall sollte ungleich Null sein, j = 2pDnL/1. Bei ausreichend großen (einige Mikrondutzende) Werten von d schwingt das Durchlässigkeitsspektrum schnell. Sollte jedoch d ca. ein µm betragen, so ist die spektrale Abhängigkeit der Durchlässigkeit ziemlich gleichmäßig, mit einer bis zwei Spitzen im sichtbaren Spektralgebiet, wobei diese Spitzen der Bedingung j = p/2+pm entsprechen. In diesem Fall gewinnt das durchgelassene Licht (in diesem Fall geht es um das Energie-Informationssignal) an "Farbe". Diese Erscheinung wird als "chromatische" Strahlungspolarisation bezeichnet. Die technische Verwirklichung dieses Effekts besteht darin, dass das Parallellichtbündel von der Lichtquelle über 2 gekreuzte Polarisatoren (Teiler) passiert. Das passierte Bündel ist dabei nicht vorhanden. Danach wird zwischen die Polarisatoren ein Kristall eingefügt. Das passierte Bündel wird gefärbt. Indem der Strahlungseinfallwinkel zur Platte geändert wird, wird die gleichmäßige Veränderung der "Farbe" des passierten (durchgelassenen) Lichtbündels erreicht. Die optische Polarisation wird zur qualitativen Schnellanalyse in der Polarisationsmikroskopie der räumlich inhomogenen anisotropen Objekte (kleiner Kristallit natürlicher Herkunft mit unregelmäßiger Geometrie, feiner Proben der ausgerichteten Flüssigkristalle und a. m.) benutzt.
(Quelle: http://www.effects.ru)

### Fachliteratur:

1. D.V. Sivukhin. Allgemeine Physik. Optik. - M.: NAUKA, 1985.
2. G.S. Landsberg. Optik. - M.: NAUKA, 1976.
3. Physik. Großes Lexikon. - M.: Große Russische Enzyklopädie, 1999 - S. 90, 460

Es ist auch bekannt, dass das Informationssignal (feines physisches Feld) beim Passieren von Gegenständen mit einer Oberflächenkrümmung die Schwingungsphase um 180 Grad verändert, d. h. invers wird. (M. Schreibmann, M. Grinstein. "Feinfeldkomponente in der Informationsmedizin" 14. Internationale Konferenz. "Theoretische und klinische Aspekte der Anwendung von Bioresonanz- und Multiresonanztherapie" - M.: IMEDIS, 2008, Band 1 - S. 79-84.)

Laut der Theorie von M.V. Kutushov lässt die Unstimmigkeit zwischen dem vorhandenen Informationsumfang und dem Informationsspeicherungsvermögen der Zelle das Vorhandensein und die Funktion von einer Art von Informationsclustern im Lebewesen annehmen. Diese Informationscluster sollten die informationshaltigen Kristallgefüge darstellen, welche noch lange Zeit nach dem Tod im Körper oder im entfernten Gewebe oder Organ erhalten bleiben (nicht zerfallen). Die spezifischen Energie-Informationsschwingungen der Informationscluster spielen anscheinend keine Rolle. Wenn sich die Substanzen mit einer bestimmten Geometrie im lebendigen Gewebe mal aufhalten lassen, prägen sie sich als Besonderheiten dieses Gewebes für eine unbestimmte Zeit ein. Aggressive Agens wie Formalin können die Information in solchen Gefügen auch nicht "löschen". Dieses Gefüge hat eine nanokristalline Herkunft und einen nanokristrallinen Aufbau. Eigentlich sind die Struktur dieser Cluster und ihr geometrischer Aufbau selbst der Speicher für die gesamte im Lebewesen angesammelte Information. Zu den Informationsclustern zählen auch die DNS-Moleküle und die Proteingefüge. Normales Protein und DNS-Moleküle schwingen mit einem bestimmten Frequenzspektrum mit. Ihre holographische Matrix resoniert mit den gleichen Frequenzen. Sogar nach dem Tod und nach der Entfernung der Gewebe aus dem Körper erzeugen sie ihre Hintergrundstrahlung weiter. Je nach der Geometrie der Gewebegefüge werden die Potenz und die Polarisationsrichtung davon unterschiedlich sein. Nach der Theorie von M.V. Kutushov gehen die sogar unwesentlich malignisierten Gewebe in höhere (Würfel, Kugel) Syngonien über. Das legt den Grad von Anisotropie und Isotropie fest. Folglich wird von den malignisierten biologischen Objekten und von den entfernten krebsbetroffenen Geweben ein nicht polarisiertes (isotropes, symmetrisches) Signal empfangen. Dementsprechend wird von gesunden biologischen Objekten und von entfernten gesunden Geweben ein polarisiertes (anisotropes, asymmetrisches) Signal empfangen (M.V. Kutushov "Krebs ist besiegbar". Herausgegeben von V. Sekatchev, 2005. S. 86-87).

Dieser Gruppe von Erfindungen liegt Folgendes zugrunde.

Allen Substanzen und Gegenständen unserer Umwelt einschließlich der biologischen Objekte ist eine gewisse sehr schwache Strahlung eigen. Diese Strahlungen sind von der Natur her Informationswellen und enthalten Informationen über die Eigenschaften des strahlenden Objekts (Strahlungsquelle). Sie werden unter die so genannten feinen physischen Felder (fine physical fields, FPF) eingereiht. Zu den Eigenschaften der Energie-Informationsstrahlung der FPF zählt das Phasenvariationsvermögen sogar bis zur Inversion, wenn diese Strahlung über den optischen Weg verläuft. Bei elektromagnetischer Strahlung kann ein solcher Effekt nur im optischen Spektralbereich beobachtet werden. Im Gegensatz dazu hängt das Phasenvariationsvermögen bei feinen physischen Feldern nicht mit ihren Frequenzkenndaten zusammen. Dabei besteht die Gleichartigkeit des polarisierten Lichts und der Strahlung von den Quellen der feinen physischen Felder darin, dass die Energie-Informationsstrahlungen ebenfalls linear polarisierte Schwingungen darstellen und dass in beiden Strahlungen das Vorhandensein von Linearpolarisation anhand von gleichen Analysengeräten erkannt werden kann. Es wurde festgestellt, dass eine der Eigenschaften der feinen physischen Felder darin besteht, dass sie unter bestimmten Bedingungen mit den Objekten der Umwelt und besonders mit biologischen Objekten, z. B. mit dem menschlichen Körper, zusammenwirken können. Diese Zusammenwirkung zeigt sich bei der Realisierung des Vorgangs der informationswellengestützten Übertragung von Eigenschaften der Quellen der FPF auf die Zwischen- oder End-Informationsträger. Dabei wird die Wellennachbildung eines einzelnen Teils der Quelle der feinen physischen Felder zum Träger von Informationswellenmerkmalen des gesamten Gegenstands. Das heißt: Jeder beliebig kleine Teil eines Trägers von feinen physischen Feldern wird die gleichen Eigenschaften haben wie die ursprüngliche Quelle. Deswegen hängt die Strahlungsintensität der flachen Quellen oder der FPF-Träger nicht von den Abmessungen (von der Größe) der strahlenden Fläche ab. Die Strahlungspolarisation ist die Ausrichtung der Wellenkraftvektoren in der Ebene, die senkrecht zum Lichtstrahl steht. Der Polarisator (Polarisationsfilter) ist ein Lichtfilter, welcher das nicht polarisierte oder teilweise polarisierte Licht in das linearpolarisierte Licht umsetzt.

Es ist Aufgabe der Erfindung, ein wirksames Verfahren und eine Einrichtung zur Umwandlung der spezifischen Energie-Informationsstrahlung von Arzneimitteln und der Energie-Informationssignale von Zellen- und Gewebegefügen sowie von anderen biologischen Objekten und die Erweiterung des Sortiments von Verfahren und Einrichtungen zur Steigerung des Nutzeffekts der bestehenden Ausrüstung für die Bioresonanztherapie und den vegetativen Resonanztest zu entwickeln.

Die gestellte Aufgabe wird durch die Merkmale des Anspruchs 1 gelöst.

Bei der Lösung der gestellten technischen Aufgabe wird folgender technische Effekt erreicht:
- eine beachtliche Steigerung des Nutzeffekts bei der Resonanzeinwirkung der Energie-Informationssignale von Arzneimitteln auf den Träger von kranken und gesunden Zellen- und Gewebegefügen,
- eine Vereinfachung des Arbeitsablaufs im Zusammenhang mit dem Auftragen der mittels Energie-Informationsstrahlung gesendeten Information auf den Träger,
- eine Verkürzung der Prozedurdauer und eine Erweiterung von Funktionsmöglichkeiten zwecks Diagnosestellung bei verschiedenen Pathologien und zwecks Bestimmung von erfolgreichen Behandlungsplänen.

Das Wesen der Erfindung in Bezug auf das vorgeschlagene Verfahren zur Umwandlung und Übertragung eines Energie-Informationssignals ist wie folgt:
Das Verfahren sieht den Empfang des superschwachen polarisierten Energie-Informationssignals (Informationswellen) von der Signalquelle sowie die Verwendung des genannten Signals zur Einwirkung auf ein biologisches oder anderes Objekt vor. Dabei wird das Energie-Informationssignal in ein Informationswellen-Bündel umgewandelt. Es wird in einen kohärenten Zustand gebracht, indem es durch wenigstens einen zwischen zwei Polarisatoren angeordneten Kristall durchgeleitet wird, damit das umgewandelte Signal nachfolgend auf das Objekt einwirkt, wobei das zwecks unmittelbarer Einwirkung oder zwecks vermittelter Einwirkung unter Einsatz von einem Zwischenträger erfolgt, welcher der Einwirkung des umgewandelten Energie-Informationssignals ausgesetzt wird.

Gemäß einer weiteren Ausgestaltung des Verfahrens hat das biologische Objekt, insbesondere der menschliche Körper, infolge der Einwirkung durch das umgewandelte Energie-Informationssignal einen um wenigstens 25 % kleineren allgemeinen biologischen Index, einen um wenigstens 20 % kleineren Fotonenindex und eine wenigstens dreifach vergrößerte Anpassungsreserve.

Gemäß einzelnen Ausgestaltungen des Verfahrens weist das Objekt infolge der Einwirkung durch das Energie-Informationssignal wenigstens um 25 % vergrößerte kennzeichnende Effektivitätskennzahlen auf.

Gemäß einzelnen Ausgestaltungen des Verfahrens wird die Einwirkung auf ein biologisches oder anderes Objekt vorgenommen, indem es die Einwirkung von einem Träger erlebt, welcher der Einwirkung des umgewandelten Energie-Informationssignals ausgesetzt worden ist.

Gemäß einzelnen Ausgestaltungen des Verfahrens dient als Quelle des ursprünglichen Energie-Informationssignals ein biologisches Objekt, insbesondere der Körper oder ein Stoff aus folgender Gruppe: ein histologisches Präparat auf einem Objektglas, Vollblut, Blutplasma oder eine biologische Flüssigkeit aus folgender Gruppe: Harn, Speichel, Sperma, Träne, Schweiß, Hautabdruck und/oder ein Stück eines Resektats oder sein Abdruck auf einem beliebigen Material.

Gemäß einzelnen Ausgestaltungen des Verfahrens wird die Einwirkung durch das umgewandelte Energie-Informationssignal von dem Objekt auf einen Informationsträger aus folgender Gruppe vorgenommen: Kapseln, ein homöopathisches Granulat, Alkohol, Wasser, Fette, Polymere, Kohlenwasserstoffe, Proteine, Metalle.

Gemäß einzelnen Ausgestaltungen des Verfahrens erfolgt die unmittelbare Einwirkung des umgewandelten Energie-Informationssignals auf den Körper zwecks Behandlung von onkologischen und /oder somatischen Erkrankungen.

Das Wesen der Erfindung und zwar der vorgeschlagenen Einrichtung ist wie folgt:
Der Energie-Informationssignalwandler enthält wenigstens einen zwischen zwei Polarisatoren angeordneten Kristall. Dabei befindet sich einer der Polarisatoren an der Seite der Quelle des Energie-Informationssignals. Der andere Polarisator ist an der Seite des durch das umgewandelte Signal beanspruchten Objekts angeordnet.

Gemäß einzelnen Ausgestaltungen ist wenigstens ein Kristall zwischen zwei gekreuzten (und /oder parallel liegenden) Polarisatoren angeordnet.

Der Wandler ist vorzugsweise mit einer Auflagefläche für das Objekt versehen. Das Objekt ist dabei eine Quelle des superschwachen Energie-Informationssignals. Dabei wird die Auflagefläche unter einem Polarisator angeordnet. Der Behälter für den Zwischenträger befindet sich oberhalb des anderen Polarisators.

Gemäß einzelnen Ausgestaltungen ist der Behälter mit einem Kabel für den Anschluss an den Stromkreis eines biophysikalischen Geräts für Bioresonanztherapie oder vegetativen Resonanztest versehen.

Gemäß einzelnen Ausgestaltungen weist der Wandler wenigstens einen Kristall aus folgender Gruppe auf: Quarzkristalle, Doppelspatkristalle, Flüssigkristalle.

Gemäß einzelnen bevorzugten Ausgestaltungen ist einer der Polarisatoren fest und der andere drehbar aufgestellt, so dass ein Drehwinkel bis 90 Grad eingestellt werden kann.

Gemäß einzelnen Ausgestaltungen enthält der Wandler zwei Kristalle, deren Achsen senkrecht zum Vektor (Strahlungsfluss) des Energie-Informationssignals zwischen den Polarisatoren liegen.

Gemäß einzelnen Ausgestaltungen ist der Wandler 180 Grad um seine eigene Horizontalachse drehbar ausgebildet, um den Bereich der Verstärkungsfaktoren von negativ bis positiv zu erweitern.

Die Erfindung wird anhand der Zeichnungen näher erläutert. Es zeigen:
- Fig. 1: ein Beispiel für die Ausführung des Energie-Informations- signalwandlers und

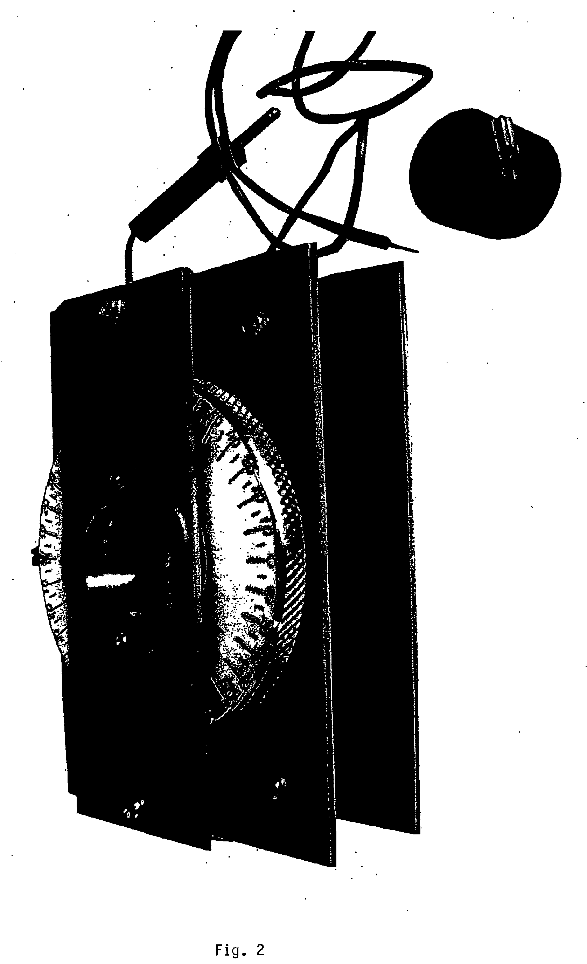

- Fig. 2: die Gesamtansicht des Energie-Informationssignalwandlers in der axonometrischen Projektion.

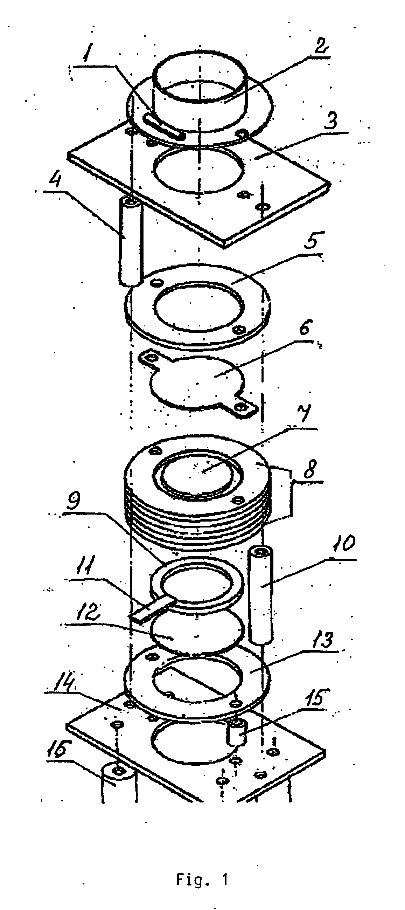

Die Bauteile in Fig. 1 sind folgerichtig getrennt dargestellt, und zwar gemäß der Signalrichtung von unten nach oben.

Der Wandler enthält einen Kontakt 1, einen Becher (Behälter) 2 für die Aufnahme des Objekts zur Einwirkung (Beanspruchung) durch ein superschwaches Energie-Informationssignal, eine obere Grundplatte 3, eine erste Stütze 4, einen Folienhalter 5, eine obere Polarisationsfolie 6 (Polarisator), eine Quarzplatte 7, einen Quarzhalter 8 (6 Stck.), einen zweiten Folienhalter 9, eine zweite Stütze 10, einen Justiermitnehmer 11, eine untere Polarisationsfolie 12 (Polarisator), einen unteren Ring 13 des Folienhalters 12, eine untere Grundplatte 14, eine dritte Stütze 15, einen Dämpfer 16 (4 Stck.), eine Auflagefläche (nicht abgebildet) für die Quelle des Energie-Informationssignals.

Der Kontakt 1 ist für die Verbindung mit einem Gerät für den vegetativen Resonanztest mit Hilfe eines Kabels vorgesehen, wenn der Energie-Informationssignalwandler in Kombination mit diesem Gerät betrieben wird. Der Becher 2 nimmt einen Behälter mit einer bestimmten Substanz (ein homöopathisches Granulat, Alkohol, Wasser usw.) auf. Diese Substanz dient zur Speicherung der verstärkten und (mit Hilfe eines Systems der Polarisatoren und Kristalle) umgewandelten Information vom Grundpräparat. Das Grundpräparat befindet sich dabei in einer Bohrung in der unteren Grundplatte 14 auf der Auflagefläche. Die obere Grundplatte 3 dient zur Befestigung des Bechers 2. Die Stützen 4, 10 und 15 (oben, mittig und unten) binden die gesamte Konstruktion der Einrichtung zusammen und sorgen für die baumäßigen Spiele zwischen dem Kristall 7 und den Polarisatoren 6 und 12. Der Quarzhalter 8 (6 Stück) ist mit runden Bohrungen für die Aufnahme der Quarzplatten 7 versehen. Jede Quarzplatte 7 ist in einem separaten Halter 8 fixiert. Die untere Polarisationsfolie 12 ist am Ring 13 des unteren Halters fixiert. Der untere Halter ist drehbar mit dem Mitnehmer 11 aufgebaut. Der Justiermitnehmer 11 sorgt für die Schwenkbarkeit des Polarisators 12, bis das maximale Signal am Kontakt 1 erreicht ist. Die Polarisatoren 6 und 12 müssen parallel sein. Sie können in einer beliebigen Ebene von horizontal bis vertikal liegen. Dabei ist der untere Ring 13 für den Folienhalter 12 (Polarisator) erforderlich, um den ersten (unteren) Polarisator 12 zu unterstützen, welcher am Kreisumfang in der horizontalen oder einer anderen Ebene bewegbar ist. In der Mitte der unteren Grundplatte 14 ist ein Rundloch ausgebildet, um darin die Auflagefläche (Schale) mit der Ausgangssubstanz (Präparat) - der Quelle des Energie-Informationssignals - zu platzieren. Der Dämpfer 16 (4 Füße) stellt den Schutz gegen Außenvibrationen und sonstige mechanische Beanspruchungen sicher.

Das vorgeschlagene Verfahren zum Auftragen der Information wird folgenderweise ausgeführt. Die Umwandlung und die Verstärkung des Energie-Informationssignals erfolgt, indem die Informationssignale durch den Energie-Informationssignalwandler durchgeleitet werden. Diese Informationssignale werden in dem Schaltkreis einer Einrichtung für Bioresonanztherapie und den vegetativen Resonanztest erzeugt und kommen von dem zu forschenden Biomaterial in den Energie-Informationssignalwandler. Dabei wird ihr Verstärkungsgrad in der für jede Art von Präparaten spezifischen Resonanzfrequenz erhöht. Als Verstärkungsanzeige dient das Rücksignal. Das Rücksignal kommt im Bioresonanztherapie-Schaltkreis auf den PC-Bildschirm und wird mit der genormten im Programm gespeicherten Antwort verglichen.

Das Signal wird zur Verstärkung durch die Information repräsentiert, welche vom Objekt auf den Informationsträger (Kapsel, homöopathisches Granulat, Alkohol, Wasser usw.) übertragen wird.

In anderen Einzelausgestaltungen ist das Biomaterial durch ein beliebiges lebendiges oder lebloses Material oder einen Stoff vertreten. Die Behandlung der erkannten onkologischen und/oder somatischen Erkrankungen erfolgt mit Hilfe eines Wandlers (Verstärkers), welcher im Schaltkreis für die Bioresonanztherapie eingeschlossen ist.

Der Energie-Informationssignalwandler kann in Form von zwei Kristallen ausgebildet sein. Einer der Kristalle ist über dem unteren unbeweglichen Polarisator und der andere Polarisator ist unter dem oberen ebenfalls unbeweglichen Polarisator angeordnet. Somit besteht die Einrichtung aus mehreren Schichten der Kristalle, gekreuzten Polarisatoren und Filtern, die in Bezug aufeinander unbeweglich (beweglich) sind.

Gemäß einzelnen Ausgestaltungen stellt der Energie-Informationssignalwandler eine Auflagefläche (Schale) für das Objekt - die Informationssignalquelle - dar. Die Auflagefläche ist unter dem unteren festen Polarisator angeordnet. Darüber befinden sich zwei Polarisatoren mit dem dazwischen angeordneten Kristall. Dabei ist einer der Polarisatoren fixiert. Der Polarisator ist am beweglichen Ring mit graduierter Skala befestigt. Oberhalb des oberen beweglichen Polarisators gibt es einen Behälter für den Informationsträger (Schale). Er ist mithilfe eines Kabels mit dem MT-Anschluss (medikamentöses Testen) des Geräts für vegetativen Resonanztest verbunden. Dabei können die Auflagefläche für die Tests sowie der Behälter aus einem beliebigen Material hergestellt sein.

Der Energie-Informationssignalwandler kann zwischen zwei gekreuzten (und/oder parallel liegenden) Polarisatoren einen beliebigen beweglich oder unbeweglich angeordneten flüssigen oder festen Kristall aufweisen (z. B. Quarz oder lslandspat). Die Achsen dieses Kristalls liegen parallel (und/oder senkrecht) zum Strahlungsvektor (Strahlungsstrom).

Als Polarisatoren können mechanische, ferritische (elektromagnetische), impulsferritische Polarisatoren aus beliebigem Material benutzt sein, welches dazu fähig ist, beliebige elektromagnetische Wellen (Energie-Informationssignale) mit beliebiger Wellenlänge und Frequenz zu polen.

Der zwischen den Polarisatoren angeordnete Kristall hat ein Kristallgitter, welches die Anisotropie der optischen Kenndaten sicherstellt.

Die Kristalle (Linse) können z. B. durch folgende Materialien vertreten werden: Kristallglas, Islandspat, Quarz, Material mit einem dispergierten Flüssigkristall oder ein anderes Material, welches die elektromagnetischen Wellen, das Licht und das Informationssignal fokussieren oder polen kann.

Gemäß einer anderen bevorzugten Ausgestaltung besteht der Energie-Informationssignalwandler aus einem unbeweglichen Teil in Form von zwei Ringen mit dazwischen angeordneten Bikonvexgläsern (Linsen), Polarisatoren, Kristallen und Invertern. Als Polarisator kann eine Folie benutzt sein. Die Folie kann aus folgenden Materialien bestehen: Turmalin, Zelluloseazetat-Schicht mit sehr vielen kleinen Herapathitkristallen (jodhaltige Verbindung von schwefelsaurem Chinin), Jod-Polyvinylfolien mit gleichartig ausgerichteten Polymerketten. Es können auch Stapel - Bunde - von feinen Plättchen isotroper Stoffe mit darauf aufgetragenen Flüssigkristallen (vertreten durch smektische, nematische und cholesterinische Flüssigkristalle) angewendet werden.

Bei der Ausführung des Verfahrens anhand der oben beschriebenen Einrichtungen zwecks präziser Diagnosestellung unterscheiden sich die superschwachen von gesunden und krebsbetroffenen Geweben und Arzneimitteln kommenden oder davon zurückgestrahlten Energie-Informationssignale mit verschiedenen Frequenzen je nach Signalcharakter. Das Signal kommt vom Ausgang des Geräts (des Verstärkers) in die Diagnose-Anlage für den vegetativen Resonanztest. Die Ergebnisse werden an der Anzeige der Resonanzskala dieser Anlage abgelesen.

Die z. B. isotropen (krebsbetroffenen) Gewebe wirken im verzerrten Modus wie "Geräusch", deswegen wird das zurückgestrahlte Rücksignal isotrop sein. Die Isotropie zeigt sich durch eine symmetrische Antwort, welche mit Hilfe des angemeldeten Verfahrens und der Einrichtung zum vegetativen Resonanztest erhalten wird. Dabei ergeben die Krebsgewebe nur ein symmetrisches Signal. Die normalen Gewebe zeigen im Gegenteil eine mehr oder weniger asymmetrische Antwort, je nach dem morphologischen, physiologischen und funktionellen Zustand.

Funktionsprinzip der vorgeschlagenen Einrichtung zur Umwandlung des Informationssignals:
Das Informationssignal kann von einem beliebigen Arzneimittel und einem biologischen Objekt (Gewebe, Blut, histologisches Präparat usw.) oder von einem biologischen Feld des Patienten empfangen werden. Das empfangene Informationssignal wird gemäß der vorliegenden technischen Lösung über einen Wandler (Verstärker) durchgeleitet.

Bei der Behandlung (Bioresonanztherapie) ist die vorgeschlagene Einrichtung nicht nur der Verstärker und Energie-Informationssignalwandler sondern auch der Inverter der Informationssignale, die im Schaltkreis der Einrichtung für Bioresonanztherapie empfangen werden.

Das physikalische Modell der vorgeschlagenen Einrichtung ist eine sog. chromatische Polarisation. Gerade bei der Betrachtung der Objektdarstellung im Polarisationsmikroskop haben ihre gleichartig gefärbten Teile eine ungefähr gleiche optische Stärke. Die Polarisationsfärbung Weiß ist eine Erscheinung, bei der das Ausgangslichtbündel durch zwei gekreuzte Polarisatoren und den dazwischen angeordneten einachsigen Kristall passiert und gefärbt wird. Die Achse des Kristalls liegt dabei senkrecht zur Lichtausbreitungsrichtung und bildet einen nicht rechten Winkel mit den Achsen der Polarisatoren.

Der durch diese Gruppe von Erfindungen vorgesehene Signalverstärkungseffekt beruht ganz und gar auf der Zustandsänderung der Strahlungspolarisation bei der Ausbreitung der Strahlung in den Kristallen und den Polarisatoren. Diese Tatsache zeugt von der elektromagnetischen Herkunft des Informationssignals.

Der Effekt der optischen Polarisation gemäß der vorgeschlagen Gruppe von Erfindungen dient zur Lösung der Aufgabe der Verstärkung der von Arzneimitteln und biologischen Objekten kommenden Informationssignale.

In der vorgeschlagenen Gruppe von Erfindungen ist das elektromagnetische (Informations-) Signal vom Testobjekt anstelle des Lichtbündels, der geregelten Kreispolarisation und der Einteilung in die rechte und linke Polarisation nicht nur der Polarisation, Bündelung und Inversion ausgesetzt, sondern auch in den kohärenten Zustand gebracht. Dadurch ist eigentlich gerade seine Verstärkung bedingt.

Während der Diagnosestellung ist die Haupteinheit der Einrichtung das Modul zum Testen von Präparaten anhand des vegetativen Resonanztests. Die Haupteinheit zur Trennung der nutzvollen und der schädlichen Informationssignale ist die vorgeschlagene Einrichtung. Der Wandler/Verstärker (ähnlich wie beim Polarisationslichtfilter) lässt die entlang der ausgesonderten Richtung polarisierte Komponente des elektromagnetischen (Informations-) Signals frei passieren und verstärkt das Informationssignal.

Während der Therapie ist in den Schaltkreis für Bioresonanztherapie der Verstärker/Wandler eingefügt. Er empfängt das vom Patienten kommende Informationssignal. Dieses Signal wird "umgekehrt", "gefärbt", "fokussiert" und/oder "invertiert". Dabei gewinnt es die erforderliche (physiologisch gesunde) Modulation und kommt in den Körper zurück.

Nach dem Eintritt dieses Signals in die Gewebe- und Zellengefüge fangen diese an, im Resonanzmodus den normalen Stoffwechsel und das normale Gefüge wiederherzustellen.

Das Informationssignal geht durch die Linse, die gekreuzten Polarisatoren und/oder den Kristall des Verstärkers/Wandlers und verstärkt das vom lebendigen und vom leblosen Material kommende Informationssignal.

In der vereinfachten Ausgestaltung ist die Polarisationseinheit nur aus einem einfachen Polarisator, einer Glaslinse oder einem einfachen festen Kristall aufgebaut. Obwohl es sich normalerweise um den in einem solchen System polarisierten Lichtstrom handelt, verfügt das sowohl von den leblosen als auch von lebendigen Objekten kommende elektromagnetische (Informations-) Signal (so die zahlreichen Testergebnisse) auch über ein Bündelungs- (Kohärenz-), Polarisations- und Verstärkungsvermögen. Gemäß den gesammelten Ergebnissen kann das Informationssignal dank der Bündelung durch die Bikonvexlinsen wie gewöhnliches Licht im sichtbaren Spektralbereich wahrgenommen sowie durch die Polarisatoren und Kristalle verstärkt und in den kohärenten Zustand gebracht werden. Dabei wird die überflüssige Hintergrundinformation (Lastinformation) in den Polarisatoren und Kristallen "abgeschnitten".

Wenn z. B. die Energie-Informationspräparate anhand des Informationssignalwandlers vorbereitet werden, so wird ihre Wirkung zuverlässig mehrmals verstärkt. Das gleiche passiert auch während der Diagnosestellung unter Einsatz der vorgeschlagenen Einrichtung im Schaltkreis für Bioresonanztherapie und vegetativen Resonanztest.

Die Vergleichsergebnisse der Behandlung des Patienten mit dem üblicherweise vorbereiteten informationshaltigen Präparat (1) und dem informationshaltigen Präparat, welches mit Hilfe des Wandlers vorbereitet worden ist, sind der Tabelle 1 zu entnehmen.

**Tabelle 1**

| Merkmal | Ausgangszustand | 1 | 2 |
|---|---|---|---|
| ABI | 10 | 7 | 4 |
| FI | 13 | 9 | 3 |
| AR | hoch 1 | hoch 4 | sehr hoch 3 |
| Skala | | | |
| ZUSTAND | 4\4 | 5\3 | 7\1 |
| Skala BG | 29 | 34 | 67 |
| Morpholog HL | 21\26 | 21\25 | 23 |
| Morpholog AF | 21\26 | 21\25 | 23 |
| Störung DNS | 4 St. | 2 St. | min. |
| Am stärksten betroffen | | | |
| Leber | bg 29 | 40 | 79 |
| Merid. LEB. | bg 34 | 45 | 81 |
| Homotox. Stad. Rekkeveg | 1\3 | 1\2 | --- |
| Leerdarm | bg 39 | 50 | 76 |
| Hämolytischer Streptokokkus | + | + | --- |
| Psor. (Sk. ZUSTAND) | 4\3 | 6\1 | 7\1 |
| Sykose | 5\2 | 6\3 | 7\3 |
| Luesinum (die erste Schäd. org.) | 7\2 | 7\3 | 7\4 |

Die Tabelle enthält folgende Merkmale:
- ABI: - allgemeiner biologischer Index
- FI: - Fotonenindex
- AR: - Anpassungsreserven
- Skala BG: - vollständige Resonanzskala des Bindegewebes
- Homotox. Stad. Rekkeveg: - Phase und Stadium von Homotoxikose nach Rekkeveg,
- Leerdarm bg: - Zustand von Leerdarm nach vollständiger Resonanz- skala beim vegetativen Resonanztest des Bindegewebes

Aus der Tabelle 1 geht hervor, dass das mit Hilfe vom Verstärker vorbereitete Präparat gegenüber dem Präparat nach dem konventionellen Verfahren die allgemeinen Kennwerte wesentlich erhöht, den Zustand der Organe mit Primärschädigungen und der am stärksten geschädigten Organe sowie des Schlüsselmeridians verbessert, Streptokokkus beseitigt und den Homotoxikose-Zustand beachtlich verbessert.

Die Arbeit zwecks Verstärkung des Energie-Informationssignals (nachfolgend Signal gleich Strahlungen) von den Pharmaka und vom biologischen Objekt (histologisches Präparat, ein Stückchen von Gewebe, Bioptat oder ihre Abdrücke auf der Gaze) wird gemäß folgender Vorgehensweise durchgeführt.

Das vom biologischen Objekt abgeleitete Informationsfeld wird auf den Träger mit Hilfe eines Magnetinduktors oder nach einem anderen allgemein anerkannten Verfahren aufgezeichnet. Der Träger wird auf die Auflagefläche unter dem Polarisator aufgestellt. Das Signal läuft durch den Wandler (nachfolgend wird auch im Text der bedeutungsgleiche Begriff Verstärker gebraucht) und kommt in die Ableitungseinrichtung ein. Dann wird es über das Kabel und den MT-Anschluss (medikamentöses Testen) an das Gerät für vegetativen Resonanztest gesendet.

Die Methode des vegetativen Resonanztests beruht auf dem Resonanz-Effekt. Der letztere entsteht während der Messung des elektrischen Hautwiderstands beim Anlegen des superschwachen konstanten elektrischen Stroms in der Größenordnung von 300 nA und während der Einführung eines zusätzlichen Informationssignals im System. Das zusätzliche Informationssignal ist dem im Körper des Patienten vorhandenen Signal ähnlich. Das Informationssignal wird mit Hilfe der vorgeschlagenen Einrichtung verstärkt und geht weiter.

Der elektrische Hautwiderstand des Menschen hängt vom Zustand des vegetativen Nervensystems ab, d. h. von dem Tonusverhältnis seines sympathischen und parasympathischen Abschnitts. Der erhöhte Hautwiderstand (Senkung des Messniveaus nach der Skala des Geräts für vegetativen Resonanztest) zeugt von der Erhöhung von Tonus des parasympathischen Abschnitts oder der Senkung von Tonus des sympathischen Abschnitts des vegetativen Nervensystems.

Das informationshaltige Präparat der Epiphyse erhöht den Tonus des Lungen-Magennerves. Bei der Belastung des Körpers mit verschiedenen Mengen an "Ampullen" des informationshaltigen Präparats der Epiphyse nimmt die Empfindlichkeit des parasympathischen Abschnitts des Nervensystems und dementsprechend der elektrische Hautwiderstand zu. Folglich, um die Empfindlichkeit zu steigern, sollte vor der Forschung eine bestimmte Menge an Ampullen der Epiphyse gleich n-1 gewählt werden. Es geht also um eine Menge, bei der noch eine weitere zusätzliche Ampulle den akuten Anstieg des elektrischen Hautwiderstands hervorruft, welcher von der Zeigerabschlag-Erscheinung auf der Skala des Geräts begleitet wird.

Die Intensität des empfangenen, den Wandler - Modulator (Verstärker) - passierten Signals wird anhand der damit mitschwingenden Potenzen der Bindegewebe-Skala ausgewertet. Dieses Bindegewebe schildert den biofunktionellen Zustand der Organe und Systeme.

Diese Einrichtung kann sowohl unabhängig als auch als ein zusätzliches Modul in der Anlage für vegetativen Resonanztest betrieben werden. Sie besteht aus einem System von Linsen, Polarisatoren und Kristallen, die unbeweglich (beweglich) übereinander angeordnet sind. Die Einrichtung enthält auch Auflageflächen unter dem unteren Polarisator zur Aufnahme der Schale mit dem Informationsträger, eine abnehmbare Vorrichtung oben mit einem Anschluss für das Verbindungskabel zum MT-Anschluss des Geräts für vegetativen Resonanztest und eine Skala (in Grad) für die Anzeige des Drehwinkels des Polaroids.

Um den erreichten Effekt und die Objektivität des Verfahrens und der Einrichtung nachzuweisen, werden folgende Beispiele angeführt.

### Beispiel 1. Patient G. 72 Jahre

Zur Übertragung der Informationen wurden die nach den konventionellen Methoden zubereiteten Träger und das Präparat angewendet. Folgende Ergebnisse wurden erreicht:
Allgemeiner Zustand des Patienten nach BG (Bindegewebe) - 22
Zustand des am stärksten betroffenen Organs - 10
Zustand des Schlüsselmeridians - 13
Phase und Stufe von Homotoxikose 3\4.
Es wurde nun die angemeldete Einrichtung (Wandler) zur Hand genommen. Das Grundpräparat wurde unter den unteren Polarisator gesetzt. An den Austritt der angemeldeten Einrichtung (des Verstärkers) wurde der Informationsträger (homöopathisches Granulat) gesetzt. 3 Minuten später wurde das auf das homöopathische Granulat aufgezeichnete Informationspräparat (informationshaltiges Präparat) mit verstärkten (gegenüber dem Grundpräparat) Informationseigenschaften erzeugt. Um die biologischen Eigenschaften des Präparats gegenüber dem Patienten zu ermitteln, wird das Präparat auf eine Platte für Medikamente im Gerät für den vegetativen Resonanztest gesetzt. Die Forschung wird am Patienten durchgeführt.

Bei der Herstellung des Präparats und der Übertragung der Information mithilfe der angemeldeten Einrichtung (des Verstärkers) mit Linsen wurden folgende Ergebnisse erreicht:
Allgemeiner Zustand nach BG - 29
Zustand des am stärksten betroffenen Organs - 20
Zustand des Schlüsselmeridians - 21
Phase und Stufe von Homotoxikose - 3\1.
Z. B.: Homotoxikose-Zustand beim Testen des mit dem Grundpräparat belasteten Patienten war 3\1 (3. Phase 1. Stadium) und bei der Belastung mit dem anhand des Verstärkers erzeugten Präparats 1/3 (1. Phase, 3. Stadium). Das ist nach Rekkeveg ein bedeutend besseres Ergebnis. Die Körperzustände können auch an der vollständigen Resonanzskala von Bindegewebe gemessen werden: Bei der Beanspruchung des Patienten mit dem Grundpräparat ergab die Untersuchung gemäß des vegetativen Resonanztests den BG - 22 Zustand. Nach der Beanspruchung mit dem erzeugten Präparat wurde der Zustand BG - 29 erreicht.

BG - Allgemeiner Zustand des Patienten nach der vollständigen Resonanzskala des vegetativen Resonanztests des Bindegewebes (100-Punktskala der Potenzen von Bindegewebe, "0" - schlecht, "100" - ideal).

### Beispiel 2. Patient G. 72 Jahre

Bei der Herstellung des Präparats und der Übertragung der Information anhand der angemeldeten Einrichtung mit zwei gekreuzten Polarisatoren wurden folgende Ergebnisse erreicht:
Allgemeiner Zustand nach BG - 33
Zustand des am stärksten betroffenen Organs - 34
Zustand des Schlüsselmeridians - 26
Phase und Stufe von Homotoxikose - 2\6.

### Beispiel 3. Patient G. 73 Jahre

Bei der Herstellung des Präparats und der Übertragung der Information anhand der angemeldeten Einrichtung mit zwei ausgelenkten Polarisatoren und zwei Flüssigkristallen wurden folgende Ergebnisse erreicht:
Allgemeiner Zustand nach BG - 43
Zustand des am stärksten betroffenen Organs - 60
Zustand des Schlüsselmeridians - 35
Phase und Stufe von Homotoxikose - 2\3.

Beispiel 4. Patient G. 74 Jahre

Bei der Herstellung des Präparats und der Übertragung der Information anhand der angemeldeten Einrichtung mit zwei ausgelenkten Polarisatoren und vier Kristallen wurden folgende Ergebnisse erreicht:
Allgemeiner Zustand nach BG - 57
Zustand des am stärksten betroffenen Organs - 68.
Zustand des Schlüsselmeridians - 53
Phase und Stufe von Homotoxikose - 2\1.

### Beispiel 5. Patient G. 72 Jahre

Bei der Herstellung des Präparats und der Übertragung der Information anhand der angemeldeten Einrichtung mit zwei ausgelenkten Polarisatoren, Quarzplatten und Flüssigkristallen wurden folgende Ergebnisse erreicht:
Allgemeiner Zustand nach BG - 60
Zustand des am stärksten betroffenen Organs - 72
Zustand des Schlüsselmeridians - 57
Phase und Stufe von Homotoxikose - 1\6.

### Beispiel 6.

Bei der Untersuchung des Patienten nach der Methode von vegetativem Resonanztest ohne Verstärker lassen sich die beim Patienten vorhandenen Toxine von Zytomegalievirus, Mononukleose, Virus von Hepatitis C, Adenovirus, Epstein-Barr-Virus, hämolytischem Streptokokkus nicht erkennen. Wird während des vegetativen Resonanztests die angemeldete Einrichtung an das Gerät für vegetativen Resonanztest angeschlossen und mit dem unter die angemeldete Einrichtung gesetzten homöopathischen Granulat versehen, welches die darin (nach einem der bekannten Verfahren) aufgezeichneten disharmonischen patientenspezifischen Schwingungen enthält, so lassen sich diese verdeckten Toxine genau erkennen.

### Beispiel 7.

Kranker mit chronischer Kolitis. Beschwerden über jahrelange periodische Schmerzen und Tenesmus. Koloskopie ergibt keine pathologischen Herde.
Test nach Standardnosode ergab keine Krebspathologie.
Es wurde ein Test in Bezug auf Symmetrie der Organpräparate mit Hilfe des Verstärkers durchgeführt. Im Blinddarm-Gebiet wurde ein Pathologiekern entdeckt. Dieser Pathologiekern zeigt sich durch das Vorhandensein von gemäßigter Isotropisierung. Das heißt, die Intensitätskennwerte des Informationssignals streben nach symmetrischer Antwort. Nachfolgend stellte sich heraus, dass der primäre Herd sich im aufsteigenden Abschnitt des Dickdarms in Form von einem 2 x 2 cm großen Polypen befand. Es wurde die Ausschneidung, des Polypen vorgenommen. Dabei wurden atypische Zellen entdeckt. Der Kranke bekam die Nosodenbehandlung, wobei die Nosoden mit Hilfe des Verstärkers im Laufe von 2 Monaten bearbeitet wurden. Eine Kontrollpolarisationsdiagnose zeigte, dass keine Metastasen vorliegen. Die CT (mit Kontrast) stellte ebenfalls keine Neubildungen fest.

Dieses Beispiel zeigt, dass während der konventionellen Untersuchung des Kranken nach der Methode des vegetativen Resonanztests keine positive Antwort auf das Vorhandensein von Malignisierung während der Beanspruchung des Patienten mit der Nosode "Carcinosium" erhalten wurde. Eine positive Antwort auf vorhandenen Krebs wurde mit Hilfe des Verstärkers erhalten, welcher in den Schaltkreis der Messung eingeschlossen wurde. Diese Antwort war die Reaktion auf die Belastung des Patienten mit dieser Nosode.

Folglich ermöglichten die anhand der angemeldeten Einrichtung verstärkten und vom Kranken abgefühlten disharmonischen Schwingungen, die Malignisierung im Dickdarmpolypen zu erkennen. Nach der Entfernung des malignisierten Polypen wurde der Kranke mit gleicher Nosode behandelt, welche ebenfalls durch die angemeldete Einrichtung bearbeitet wurde. Anhand der angemeldeten Einrichtung wird die Verstärkung der Informationskomponente von sowohl homöopathischen Präparaten als auch von Nosoden und den Informationsfeldern vorgenommen. Die Informationsfelder wurden während der Bioresonanztherapie des Kranken an verschiedenen Stoffen und Objekten der belebten und unbelebten Natur abgefühlt.

### Beispiel 8.

Der Kranke mit chronischer Lungenentzündung wurde dem vegetativen Resonanztest mit einem Polarisator ausgesetzt. Die Dissymmetrie ist nicht ausgeprägt. Der obere Polarisator ist um 90 Grad zum unteren versetzt. Die Polarisationsanzeige ist jedoch in einer solchen Lage 5 rechts und 7 links. Es liegt also eindeutig eine mäßige Dissymmetrie vor. Sie zeugt von der chronischen Lungenentzündung mit einem Malignisierungstrend. Es wurde ein ergänzender Test mit Hilfe des Signalverstärkers durchgeführt. Als Antwort wurde ein symmetrisches Signal empfangen. Dies spricht für eine vorhandene Krebspathologie. Nachfolgende Untersuchungen (CT und Bronchoskopie) bestätigten das Vorhandensein von bronchopulmonalem Karzinom in der rechten Lunge. Es wurde die dissymmetriegestützte Therapie (DST) durchgeführt (Inhalationen, Getränke und Mikroklistiere). Dabei wurden die DST- Präparate der Behandlung anhand des Verstärkers mit einer Linse und Kristallen ausgesetzt. Das Husten wurde innerhalb von zwei Monaten beseitigt. CT und Bronchoskopie zeigen keine Bildungen.

Bei dem vorläufigen vegetativen Resonanztest wird der obere Polarisator der angemeldeten Einrichtung um 90 Grad zum unteren versetzt. Das bringt eine minimale Signalverstärkung. Bei einer solchen Anordnung der Polarisatoren wurde ein geringfügiger Unterschied zwischen der Abweichung des Polarisators der angemeldeten Einrichtung von 90 Grad nach rechts und nach links während der Tests nach der vollständigen Resonanzskala von Bindegewebe festgestellt (5 und 7, d. h. insgesamt um 2 Punkte). Eine solche Dissymmetrie zeugt von einem chronischen Entzündungsvorgang mit Verdacht auf Malignisierung. Liegt der Unterschied zwischen den Werten der Bindegewebe-Skala unter 7 Punkten, wobei die Polarisatoren der angemeldeten Einrichtung in horizontaler Ebene um 90 Grad zueinander geschwenkt sind, sollte die Tendenz zum Malignisierungsvorgang angenommen werden. DST steht für eine dissymmetriegestützte Therapie. Das ist eine neue Methode für die Vorbeugung und die Behandlung von onkologischen Erkrankungen. Die in der dissymmetriegestützten Therapie benutzten Präparate werden zurzeit noch erforscht. Doxorubicinum (Doxorubicin) ist ein pharmakologisches Präparat, welches bei der Chemotherapie zur Behandlung von onkologischen Krankheiten angewendet wird. Skala in Grad ist eine Skala zur Anzeige des Drehwinkels des Polarisators. Jede Teilung dieser Skala entspricht 5 Grad.

### Beispiel 9.

Das DST-Präparat ist auf das Objektglas aufgetragen und auf die Auflagefläche zum Testen gesetzt. Die Lage des Polarisators der angemeldeten Einrichtung entspricht 5 Grad rechts. Das Signal vom Präparat zeigt das Übereinstimmen mit Adenokarzinom von Vorsteherdrüse, Milchdrüse, Magen, Lungen. Der Polarisator steht auf 5 Grad links. Das Signal vom Präparat zeigt ein Übereinstimmen mit denselben Arten von Adenokarzinom an. Auf die Ablage für die Testpräparate wurde das Präparat Doxorubicinum gesetzt. Der Polarisator wurde auf 5 Grad rechts eingestellt. Das Signal vom Präparat zeigte kein Übereinstimmen mit Adenokarzinom von Vorsteherdrüse, Milchdrüse, Magen, Lungen an. Der Polarisator wurde auf 5 Grad links eingestellt. Das Signal vom Präparat zeigte kein Übereinstimmen mit denselben Arten von Adenokarzinom an. Der Signalverstärker wurde vor dem Polarisator angeordnet. Es wurde die zuverlässige Bestätigung der Präparate mit Adenokarzinom von Vorsteherdrüse erhalten. Erfahrungsgemäß wurde festgestellt, dass Symmetrie (z. B. BG Potenz 5 rechts und BG Potenz 5 links) oder schwache Dissymmetrie (mit Unterschied unter 7 Punkten) beim Schwenken des oberen Polarisators gerade um 5 Grad nach rechts und nach links von der 90-Grad- Stellung (gegenüber dem unteren Polarisator) während des vegetativen Resonanztests mit Resonanzskala von Bindegewebe erkannt wird. Das zeugt von einem vorhandenen onkologischen oder voronkologischen Zustand. Wird der vegetative Resonanztest des gesunden Menschen durchgeführt, indem er mit den Präparaten (Nosoden) von Vorsteherdrüse-, Milchdrüse-, Magenkrebs usw. belastet wird, und der Polarisator um 5 Grad nach rechts und nach links gedreht wird, liegt Symmetrie vor (Potenz der BG-Skala 5 und 5). Das heißt, das Vorhandensein von Krebs mit bestimmter Nosologie (gemäß der Tumornosode, die auf die Medikamentenplatte zur Beanspruchung während des vegetativen Resonanztests gesetzt worden ist) wird bestätigt. Sollte gleichzeitig mit der Tumornosode ein antiblastomatöses Präparat auf die Medikamentenplatte gesetzt werden, so kann festgestellt werden, ob dieses Präparat bei der Behandlung dieses Tumors wirksam ist.

Es werden 2 antiblastomatöse Präparate erforscht: DST und Doxorubicinum. Bei der Belastung mit dem DST-Präparat in Kombination mit Nosoden von Tumoren mit verschiedener Ortung wurde eine Resonanz nach der Bindegewebe-Skala beobachtet. Bei der Belastung mit Doxorubicinum wurde die Resonanz erst nach der Verstärkung der Informationskomponente des Präparats anhand der angemeldeten Einrichtung erzielt. Somit wurde festgestellt, dass diese Art von onkologischer Krankheit mit den DST-Präparaten oder mit dem verstärkten Präparat Doxorubicinum behandelt werden kann. Das übliche Doxorubicinum wird dabei nutzlos sein.

### Beispiel 10.

Der Patient mit Diagnose Hochdruckkrankheit Stadium 2B wurde nach dem vegetativen Resonanztest untersucht. Hinweis auf die Hochdruckkrankheit ist positiv. Allgemeiner Zustand nach der ZUSTAND-Skala - 1\1.

Aufgrund dieses niedrigen Wertes Verdacht auf einen onkologischen Vorgang. Um die Onkologie zu prüfen, wurde der Test unter Einsatz der angemeldeten Einrichtung durchgeführt. Auf die Medikamentenplatte wurde ein Fläschchen mit dem DST- Präparat gesetzt.

Kennwert "ZUSTAND" wurde nun 4\3. Das zeugt von der vorhandenen Onkologie. Während der nachfolgenden eingehenden Untersuchung wurde Phäochromozytom festgestellt. Während des vegetativen Resonanztests werden verschiedene Diagnoseskalen eingesetzt (Resonanzskala von Bindegewebe, "ZUSTAND"-Skala 7-Punktresonanzskala, jeder Punkt hat noch 3 bis 4 Abstufungen). Sollte der allgemeine Zustand des Patienten nach dieser Skala getestet werden, so ist der Zustand des Patienten sehr schlecht bei Anzeige "1\1 " des Informationspräparats dieser Skala und ideal bei Anzeige "7\4". Wird der von keinem onkologischen Prozess betroffene Patient mit einem antiblastomatösen Präparat belastet und nach der "ZUSTAND"-Skala getestet, so wird keine Verbesserung der Merkmale nach dieser Skala erreicht. Bei vorliegender Onkologie wird während der Belastung des Kranken mit dem antiblastomatösen und für diesen Patienten wirksamen Präparat, z. B. DST, Verbesserung nach der "ZUSTAND"-Skala (in diesem Beispiel - 4\3) beobachtet. Diese Tatsache bestätigt den vorhandenen onkologischen Vorgang.

### Beispiel 11.

Patient mit Fieber und einem Hyperämieherd auf dem Fuß. Auf die Auflagefläche wurde ein Prüfglas (1 ml Behälter) mit der Kultur von hämolytischem Streptokokkus zum Testen gesetzt. Beim vegetativen Resonanztest ergab es eine wenig ausgeprägte positive Reaktion. Der Nosodetest der Erysipelentzündung war negativ. Nachdem das Informationssignal der Nosode durch die angemeldete Einrichtung durchgeleitet worden war, wurde ein präzises Resonanzsignal als Antwort auf ämolytischen Streptokokkus und Erysipelentzündung empfangen. Die Entzündung wurde durch die häufige Einnahme von Präparat Lachesis 12 innerhalb von einigen Stunden kupiert.

### Beispiel 12.

Die angemeldete Einrichtung (Verstärker, Inverter, Wandler) kann zur Behandlung von Krebs sowie von anderen Pathologien eingesetzt werden, indem sie in den Schaltkreis für Bioresonanztherapie eingeschlossen wird. So bekam eine Patientin mit Mammakarzinom und Metastasen in den Achsel-Lymphknoten 10 Behandlungen nach der Bioresonanztherapie mit Verstärker (Inverter, Wandler) der Informationssignale. In diesem Fall kehrten die Linse, der Kristall und die Polarisatoren das zurückkommende Informationssignal in den Körper um. Dabei wurde keine medikamentöse und sonstige ergänzende Behandlung der Patientin vorgenommen. Nach 1,5 Monaten wurde der ursprüngliche Herd um 70 % kleiner. Achsel-Lymphknoten lassen sich nicht abfühlen. Vor der Behandlung nach der Bioresonanztherapie-Methode und mit Hilfe der angemeldeten Einrichtung (Wandler, Inverter) war der Tumor schmerzhaft und unbeweglich. Während der nachfolgenden Behandlung im Laufe von 3 Monaten hörte das Wachstum des Tumors auf und seine Abmessungen verringerten sich bis zu 1,5 cm. Darüber hinaus wurde der Tumor weich und schmerzlos. Achsel-Lymphknoten lassen sich nicht abfühlen. Der vegetative Resonanztest mit einem Polarisator zeigte eine stark ausgeprägte Dissymmetrie an. Die Antikrebs-Resistenz änderte sich von sehr niedrig auf hoch. Das Onkoprotein betrug D100 (statt Theta). Die Anpassungsreserven wurden als "gut" (4) gegen die ursprünglichen "erschöpft" (2) bewertet. Anstelle des 3. klinischen Stadiums wurde das 1. festgestellt.

### Beispiel 13.

Der Kranke mit Diagnose chronische destruktive Lungenentzündung bekam 20 Behandlungen nach der Bioresonanztherapie unter Einsatz der angemeldeten Einrichtung (Verstärker, Wandler, Inverter). Während der Therapie sind folgende Symptome verschwunden: Husten, Schmerzen im Brustkorb und Sputum. Drei Monate nach Beginn der Behandlung wurde die CT durchgeführt. Sie zeigte eine deutliche Verbesserung des Gewebegefüges in den Lungen und die Verringerung der Paraaorten- und Paratrachealymphknoten.

Somit wurden ein wirksames Verfahren und eine Einrichtung zur Verstärkung, Umwandlung und Inversion (Umkehrung) des Informationssignals sowohl von biologischen Objekten als auch von Arzneimitteln und Trägern entwickelt. Dabei wurden die Zuverlässigkeit der Verstärkung des Informationssignals erhöht, die Vorgehensweise beim Auftragen der Informationen auf den Träger vereinfacht und die Behandlungsdauer bei der Behandlung von unterschiedlichen Pathologien, darunter auch Krebs, verkürzt.

### Beispiel 14.

Patientin mit chronischer Hepatitis.
Ausgangszustand - 2\1 ,
nach der BG- Skala - 17,
das am stärksten betroffene Organ - Leber - BG-8,
Leberzustand nach Homotoxikose-Phasen - 3\5.
Es wurde die standardisierte Grund-Bioresonanztherapie vorgenommen. Danach wurden folgende Ergebnisse erhalten:
Zustand - 5\1 ,
nach der BG- Skala - 38,
Leber - BG -14,
nach Homotoxikose-Phasen - 2\3.

Nach der direkten Grund-Bioresonaztherapie mit Hilfe der angemeldeten Einrichtung wurden die Merkmale beachtlich besser:
Zustand 7\2,
nach der BG- Skale - 72,
Leber - BG -46,
nach Homotoxikose-Phasen - 1\2.

Beim vegetativen Resonanztest werden verschiedene Diagnoseskalen eingesetzt (Resonanzskala von Bindegewebe, "ZUSTAND"-Skala 7-Punktresonanzskala, jeder Punkt hat noch 3 bis 4 Abstufungen). Sollte der allgemeine Zustand des Patienten nach dieser Skala getestet werden, so ist der Zustand des Patienten sehr schlecht bei einer Anzeige "1\1" des Informationspräparats dieser Skala und ideal bei einer Anzeige "7\4". Wird der von keinem onkologischen Prozess betroffene Patient mit einem antiblastomatösen Präparat belastet und nach der "ZUSTAND"-Skala getestet, so wird keine Verbesserung der Merkmale nach dieser Skala erreicht. Bei vorliegender Onkologie wird während der Belastung des Kranken mit dem antiblastomatösen und für diesen Patienten wirksamen Präparat, z. B. DST, Verbesserung nach der "ZUSTAND"-Skala (in diesem Beispiel - 4\3) beobachtet. Das gilt als Nachweis für den vorhandenen onkologischen Vorgang.

### Beispiel 15.

Dem Kranken mit der Diagnose Hochdruckkrankheit 3. St. wurde ein drucksenkendes Mittel aus der Gruppe von Betablockern verordnet, und zwar je 2 Pillen (0,50 mg) im Laufe von 2 Monaten. Der arterielle Blutdruck betrug vor der Behandlung 240/110 mm Hg und nahm im Laufe von einem Monat bis auf 160/95 mm Hg ab. Während des zweiten Monats blieb der arterielle Blutdruck ungefähr auf gleichem Niveau. Um den therapeutischen Effekt zu verstärken, wurde das gleiche Präparat durch den Verstärker der Informationssignale bearbeitet. Dem Kranken wurde derselbe Betablocker verordnet, jedoch mit fünfmal kleinerer Dosis. Innerhalb der ersten zwei Wochen sank der Blutdruck bis 145/95 mm Hg. Danach wurde eine absteigende Drucksenkung beobachtet. Im Laufe von zwei Monaten wurde der arterielle Blutdruck stabil und betrug 130/80 - 125/75 mm Hg. Folglich gewinnt das konventionelle allopathische Präparat nach der Einwirkung durch den Verstärker an erhöhten drucksenkenden Eigenschaften. Es geht also um die Qualitätserhöhung der allopathischen Mittel, bei denen für einen Patienten nicht nur die chemische Zusammensetzung sondern auch die Informationskomponente von Bedeutung sind. So wird in diesem Fall 1 Pille des Ursprungsmaterials unter den Verstärker und die für die Therapie erforderliche Pillenmenge an den Ausgang des Wandlers (des Verstärkers) gesetzt. Nach der Einwirkung bekommt man Präparate mit einer verbesserten Informationskomponente.

### Beispiel 16.

Ein Kranker mit Diagnose chronische destruktive Bronchopneumonie in akuter Form. Ausgeprägte Atemnot, Schmerzen im Brustkorb und schweres Husten. Das Röntgenbild zeigt die typische Bronchopneumonie. Verordnung: Antibiotikum "Abaktal" (Pefloxacin) à 1 Pille zweimal täglich. Solche Verordnung wurde dadurch bedingt, dass der Patient allergische Reaktion gegen alle Antibiotika bis auf Abaktal zeigt. Darüber hinaus zeigte sich Abaktal auch als das wirksamste Mittel beim Testen auf Empfindlichkeit gegen Antibiotika. Husten, Schwäche und Atemnot ließen zwar nach, aber sie verschwanden nicht. Die Antibiotika wurden 10 Tage lang eingenommen. Um den therapeutischen Effekt zu verstärken, wurde das gleiche Antibiotikum durch den angemeldeten Wandler (Verstärker) der Informationssignale durchgeleitet. Zwei Wochen später wurde dem Kranken dasselbe Präparat verordnet, jedoch in zehnfach kleinerer Dosis. In den ersten Wochen ließen Husten und Schmerzen im Brustkorb sofort nach. Innerhalb von zwei Monaten hörte das Husten ganz auf und die Atemnot ließ beachtlich nach. Das Röntgenbild zeigt gut gestaltetes Lungengefüge. Die Lungenfelder sind klar.

Somit gewinnt ein übliches Antibiotikum nach der Behandlung durch den Verstärker erhöhte antimikrobielle Eigenschaften.

### Beispiel 17.

Dem Kranken mit Diagnose chronische Kolitis wurde ein Nahrungsergänzungsmittel mit ausgeprägter Abführwirkung verordnet: à 4 Kapseln pro Tag (1 Kapsel enthält 150 mg Wirkstoffe) im Laufe von 1 Monat. Es wurde praktisch kein ausgeprägter Abführeffekt erreicht. Um den therapeutischen Effekt zu verstärken, wurde das gleiche Nahrungsergänzungsmittel durch den angemeldeten Wandler (Verstärker) der Informationssignale durchgeleitet. Danach wurde dem Kranken dasselbe Präparat verordnet, jedoch sollte er nur 1 Kapsel pro Tag einnehmen. Der ausgeprägte Abführeffekt wurde bereits in den ersten Tagen festgestellt. Im Laufe von zwei Wochen wurde der Stuhl normal. Der nachfolgende Verzicht auf die Einnahme von Nahrungsergänzungsmitteln hatte keine Auswirkungen auf die Defäkation.

Somit bekommt ein übliches für die Stuhlnormalisierung vorgesehenes Nahrungsergänzungsmittel nach der Behandlung im Verstärker verbesserte Abführeigenschaften.

### Beispiel 18.

Behandlung von Treibstoff - Benzin 95. Eine 2 ml große Schale wurde mit Benzin gefüllt und in die zweite Schale des Geräts für Bioresonanztherapie gesetzt. Auf die erste Schale dieses Geräts wurde der angemeldete Wandler (Verstärker) aufgestellt. Eine weitere 2 ml große Schale mit gleichem Benzin wurde am Ausgang des Verstärkers angeordnet. Das unabhängige Gerät für Bioresonanztherapie wurde gestartet. Im Endeffekt wurde in der Schale am Ausgang des Verstärkers das Benzin mit verbesserten Eigenschaften hergestellt. 2 ml dieses Benzins wurden nachher in den Benzintank des Autos eingegossen. Die Information vom Benzin mit verbesserter Qualität aus der Schale breitete sich über die ganze Benzinmenge im Tank aus. Mit diesem Benzin konnte das Auto 35 km mehr fahren als üblich.

### Beispiel 19.

Behandlung der Lebensmittel - Rotwein. 2 ml Wein wurde in die Schale eingeschenkt und in die zweite Schale des Geräts für Bioresonanztherapie gesetzt. Auf die erste Schale dieses Geräts wurde der angemeldete Wandler (Verstärker) aufgestellt. Eine weitere Schale mit 2 ml gleichen Weins wurde am Ausgang des Verstärkers angeordnet. Das unabhängige Gerät für Bioresonanztherapie wurde gestartet. Die Therapie wurde an allen Meridianen durchgeführt. Nach der Behandlung wurde in der Schale am Ausgang des Verstärkers der Wein mit verbesserten Geschmackseigenschaften erzeugt. Diese 2 ml Wein wurden in die Weinflasche eingegossen und durchgeschüttelt. Der ganze Wein in der Flasche bekam einen angenehmeren Geschmack. Die organoleptischen Eigenschaften wurden verbessert.

Demnach wird die Qualität der Therapie beim Anschluss des Verstärkers am Ausgang des direkt betriebenen Geräts für Bioresonanztherapie wesentlich erhöht. Jedes Mal, wenn das aus dem Gerät für Bioresonanztherapie ausgehende Signal das System von Polarisatoren und Kristallen des angemeldeten Wandlers passiert, wird dieses Signal verstärkt, moduliert und harmonisiert. Dadurch kann der Körper die Anpassungssysteme zur Selbstorganisation aller Homöostase-Systeme effizienter bringen. Somit wurden das wirksame Verfahren und die Einrichtung zur Verstärkung, Umwandlung und Invertierung des Energie-Informationssignals sowohl von biologischen Objekten als auch von Arzneimitteln, Lebensmitteln und Trägern entwickelt. Das biologische Objekt, insbesondere der menschliche Körper, hat infolge der Einwirkung durch das umgewandelte Energie-Informationssignal einen um wenigstens 25 % kleineren allgemeinen biologischen Index, einen um wenigstens 20 % kleineren Fotonenindex und wenigstens eine um das Dreifache vergrößerte Anpassungsreserve. Das Produkt weist wenigstens um 25 % vergrößerte kennzeichnende Effektivitätskennzahlen auf. Dabei ist die Zuverlässigkeit der Informationssignalverstärkung erhöht. Der Arbeitsablauf im Zusammenhang mit dem Auftragen der Information auf den Träger ist vereinfacht und die Behandlungsdauer verschiedener Pathologien, darunter auch von Krebs, ist verkürzt.

## Patentansprüche

1. Verfahren zur Umwandlung und Übertragung eines Energie-Informationssignals, bei dem ein superschwaches polarisiertes Energie-Informationssignal (Informationswellensignal) von einer Signalquelle empfangen wird, und bei dem das genannte Signal zwecks Einwirkung auf ein biologisches oder ein anderes Objekt verwendet wird,
**dadurch gekennzeichnet,**
**dass** das Energie-Informationssignal zu einem Informationswellenbündel umgewandelt wird, wobei das Informationswellenbündel in einen kohärenten Zustand gebracht wird, indem es über wenigstens einen zwischen zwei Polarisatoren angeordneten Kristall durchgeleitet wird, damit das umgewandelte Signal nachfolgend auf das Objekt einwirkt, unter anderem zwecks unmittelbarer Einwirkung oder zwecks vermittelter Einwirkung unter Einsatz von einem Zwischenträger, welcher durch das umgewandelte Energie-Informationssignal beeinflusst wird.

2. Verfahren nach Anspruch 1,
**dadurch gekennzeichnet,**
**dass** das biologische Objekt, insbesondere der menschliche Körper, infolge der Einwirkung durch das umgewandelte Energie-Informationssignal einen um wenigstens 25 % kleineren allgemeinen biologischen Index, einen um wenigstens 20 % kleineren Fotonenindex und eine wenigstens dreifach vergrößerte Anpassungsreserve aufweist.

3. Verfahren nach Anspruch 1,
**dadurch gekennzeichnet,**
**dass** das Objekt infolge der Einwirkung durch das Energie-Informationssignal eine wenigstens um 25 % vergrößerte kennzeichnende Effektivitätskennzahl aufweist.

4. Verfahren nach Anspruch 1,
**dadurch gekennzeichnet,**
**dass** das biologische oder das andere Objekt die Einwirkung von einem Träger erlebt, welcher der Einwirkung des umgewandelten Energie-Informationssignals ausgesetzt wird.

5. Verfahren nach Anspruch 1,
**dadurch gekennzeichnet,**
**dass** als Quelle des ursprünglichen Energie-Informationssignals ein biologisches Objekt und insbesondere der Körper oder ein Stoff aus folgender Gruppe verwendet wird: ein histologisches Präparat auf einem Objektglas, Vollblut, Blutplasma oder eine biologische Flüssigkeit aus folgender Gruppe: Harn, Speichel, Sperma, Träne, Schweiß, Hautabdruck und/oder ein Stück eines Resektats oder sein Abdruck auf einem beliebigen Material.

6. Verfahren nach einem der Ansprüche 1, 3, 4 und 5,
**dadurch gekennzeichnet,**
**dass** die Einwirkung des umgewandelten Energie-Informationssignals vom Objekt auf einen Informationsträger aus folgender Gruppe vorgenommen wird: Kapseln, homöopathisches Granulat, Alkohol, Wasser, Fette, Polymere, Kohlenwasserstoffe, Proteine, Metalle.

7. Verfahren nach Anspruch 1 und 2,
**dadurch gekennzeichnet,**
**dass** die unmittelbare Einwirkung des umgewandelten Energie-Informationssignals auf den Körper zwecks Behandlung von onkologischen und/oder somatischen Erkrankungen vorgenommen wird.

8. Energie-Informationssignalwandler mit wenigstens einem zwischen zwei Polarisatoren angeordneten Kristall, wobei einer der Polarisatoren an der Seite einer Quelle eines Energie-Informationssignals und der andere Polarisator an der Seite eines durch das umgewandelte Signal beanspruchten Objekts angeordnet sind.

9. Energie-Informationssignalwandler nach Anspruch 8,
**dadurch gekennzeichnet,**
**dass** wenigstens ein Kristall zwischen zwei gekreuzten (und/oder parallel liegenden) Polarisatoren angeordnet ist.

10. Energie-Informationssignalwandler nach Anspruch 9,
**dadurch gekennzeichnet,**
**dass** er mit einer Auflagefläche für das Objekt, welches eine Quelle eines superschwachen Energie-Informationssignals bildet und mit einem Behälter für den Zwischenträger versehen ist, wobei die Auflagefläche unter einem Polarisator und der Behälter für den Zwischenträger oberhalb des anderen Polarisators angeordnet sind.

11. Energie-Informationssignalwandler nach Anspruch 9,
**dadurch gekennzeichnet,**
**dass** der Behälter mit einem Kabel für den Anschluss an einen Stromkreis eines biophysikalischen Geräts aus dem Fachbereich Bioresonanztherapie oder vegetativer Resonanztest versehen ist.

12. Energie-Informationssignalwandler nach einem der Ansprüche 8 bis 11,
**dadurch gekennzeichnet,**
**dass** er wenigstens einen Kristall aus folgender Gruppe enthält: Quarzkristalle, Doppelspatkristalle, Flüssigkristalle.

13. Energie-Informationssignalwandler nach einem der Ansprüche 8 bis 11,
**dadurch gekennzeichnet,**
**dass** einer der Polarisatoren fest angeordnet ist und der andere drehbar aufgestellt ist, so dass ein Drehwinkel bis 90 Grad einstellbar ist.

14. Energie-Informationssignalwandler nach einem der Ansprüche 8 bis11,
**dadurch gekennzeichnet,**
**dass** er zwei Kristalle aufweist, deren Achsen senkrecht zum Vektor (Strahlungsfluss) des Energie-Informationssignals zwischen den Polarisatoren stehen.

15. Energie-Informationssignalwandler nach einem der Ansprüche 8 bis 11,
**dadurch gekennzeichnet,**
**dass** er 180 Grad um seine eigene Horizontalachse drehbar ausgebildet ist, wobei eine Erweiterung des Bereichs der Verstärkungsfaktoren von negativ bis positiv ermöglicht ist.
